# EUROPÄISCHE PATENTSCHRIFT

(11) **EP 1 671 765 B1**
(45) Veröffentlichungstag und Bekanntmachung des Hinweises auf die Patenterteilung: **23.05.2007**
(21) Anmeldenummer: 04030045.1
(22) Anmeldetag: 17.12.2004
(51) Int. Cl.: B27K 3/00, A47G 9/00, A47C 27/12, B27L 11/00

(54) **Zirbenholzpräparat und Verfahren zu seiner Herstellung**
Swiss stone pine preparation and process for production
Préparation de pin arolle et procédé de fabrication

(43) Veröffentlichungstag der Anmeldung: 21.06.2006
(73) Patentinhaber: Dryer Systems Trocknerbau GmbH, 6330 Kufstein (AT); Girardelli, Erika, 6330 Kufstein (AT)
(72) Erfinder: Giradelli, Dagobert, 6330 Kufstein (AT)
(74) Vertreter: Wunderlich, Rainer

(56) Entgegenhaltungen:
- WO-A-97/43053
- DE-A1- 19 513 997
- DE-A1- 19 626 380
- GB-A- 2 210 555

## Beschreibung

Die Erfindung betrifft ein Zirbenholzpräparat und ein Verfahren zu dessen Herstellung.

Die Zirbelkiefer (lateinisch: pinus cembra) ist ein Baum, welcher in Hochgebirgslagen, insbesondere der Alpen und Karpaten, vorkommt. Aufgrund der extremen Witterungsverhältnisse ist die Zirbelkiefer extrem widerstandsfähig, wobei der relativ langsam wachsende Baum bis zu 1000 Jahre und darüber hinaus alt werden kann.

Das Holz der Zirbelkiefer, das sogenannte Zirbenholz, wird seit vielen Jahrhunderten für Möbel sowie Decken- und Wandvertäfelungen eingesetzt. Mit Zirbenholz gestaltete Zimmer, sogenannte Zirbelstuben, werden seit Jahrhunderten für ihre angenehme und wohltuende Atmosphäre geschätzt. Neben einer besonderen Resistenz gegen Bakterien- und Schädlingsbefall wird insbesondere auch der als angenehm und wohltuend empfundene Geruch als vorteilhaft geschätzt.

Es ist bekannt, aus den Nadeln und Zapfen der Zirbelkiefer das sogenannte Zirbelöl zu gewinnen. Dieses Zirbelöl enthält ätherische Öle sowie eine Vielzahl wertvoller Inhaltstoffe, deren positive Wirkungen und Eigenschaften seit langer Zeit für die Gesunderhaltung der Menschen eingesetzt werden.

In der GB -A- 2 210 555 A wird ein Verfahren zur Herstellung einer Polsterfüllung aus kugelförmigen, mit einer aufgerauten Oberfläche versehenen Holzpartikeln offenbart. Als Material wird vorzugsweise das Holz der japanischen Zypresse oder alternativ ein anderes beliebiges Holz gewählt, welches dann mit einer duftenden, öligen Essenz der japanischen Zypresse behandelt wurde.

In der DE -A- 196 26 380 wird für eine gesunde Schlafbequemlichkeit die Materialzusammensetzung für eine Zudecke bzw. Schlafunterlage beschrieben. Als Füllung werden die Naturprodukte Baumwolle, kalt gewaschene, kardierte Schafwolle, Kamelhaar, Hobelspäne, Dinkelspitzen bzw. Maislieschen vorgeschlagen, die auch mit Kräutern vermischt werden können. Dabei kann ein Kissen aus zwei Kissenbezügen mit voneinander verschiedenen Naturprodukten als Füllmaterialien bestehen.

In der DE -A- 195 13 997 ist ein in der Höhe verstellbares Nackenkissen offenbart, welches als ein Füllmaterial unter anderem Holzspäne aufweisen kann.

In der WO -A- 97/43053 ist ein Verfahren und eine Anordnung zum Herstellen von Tierbettzeug aus wiederverwendeten Papierabfall beschrieben, der mit chemischen Zusätzen versetzt werden kann.

Der Erfindung liegt die **Aufgabe** zugrunde, ein Präparat aus Zirbenholz und ein Verfahren zu dessen Herstellung anzugeben, mit welchen die positiven Wirkungen, insbesondere der wohltuende Duft der Zirbelkiefer besonders effizient und langfristig bereitgestellt werden können.

Die Aufgabe wird durch ein Verfahren mit den Merkmalen des Anspruchs 1 und mit einem Zirbelholzpräparat mit den Merkmalen des Anspruchs 6 gelöst. Bevorzugte Ausführungsformen sind in den abhängigen Ansprüchen angegeben.

Ein erfindungsgemäßes Verfahren zur Herstellung eines Zirbenholzpräparates wird dadurch erreicht, dass Zirbenholz zu Spänen zerkleinert wird, welche anschließend mit Zirbenöl imprägniert werden.

Die Erfindung beruht auf der Erkenntnis, dass im Holz der Zirbelkiefer die ätherischen Öle sowie die positiven Inhaltstoffe in einem ganz besonderen Maße enthalten und gespeichert sind. Durch Zerkleinerung des Zirbenholzes in Späne wird eine relativ große Oberfläche geschaffen, so dass die Duftstoffe besonders gut abgegeben werden können. Dabei ist es erfindungsgemäß, dass die Zirbenholzspäne zusätzlich mit Zirbenöl imprägniert sind. Dieses Zirbenöl kann in bekannter Weise aus den Nadeln, Zapfen oder Holzbestandteilen der Zirbelkiefer gewonnen sein.

Es wurde festgestellt, dass bei den erfindungsgemäß behandelten Zirbenholzspänen der Geruch nicht nur als besonders intensiv, angenehm und wohltuend empfunden, sondern auch über eine lange Zeitspanne nahezu gleich bleibend wahrgenommen wird. Als eine mögliche Erklärung kann angesehen werden, dass das aufgebrachte Zirbenöl die im Holz befindlichen Duft- und Inhaltstoffe zusätzlich aufschließt und deren Freisetzung verbessert. Gerade bei Zirbenöl aus den Nadeln ist durch die Rückführung und Zusammenbringung der Inhaltstoffe aus den äußeren Baumbereichen, nämlich den Nadeln, zu den im Innern des Baumes verbliebenen und gespeicherten Inhaltstoffen von einer besonderen Wechselwirkung und geradezu von einer gegenseiten Potenzierung der Vielzahl der einzelnen Duft- und Wirkstoffe auszugehen.

Das so hergestellte Zirbenholzpräparat kann für vielfältigste Anwendungszwecke eingesetzt werden, insbesondere zur Raum- und Klimaverbesserung sowie zu pharmazeutischen und kosmetischen Zwecken. Insbesondere bei stressbedingten Beschwerden, wie Nervosität, gestörter Blutdruck und Herzfrequenz sowie Schlaf- und Essstörungen, konnten ganz erhebliche Bessungen und Linderungen bei Menschen festgestellt werden. Dies ist auf die beruhigende und entspannende Wirkung der Duft- und Wirkstoffe des Präparats zurückzuführen.

Unter Imprägnieren im Sinne der Erfindung ist sowohl ein Besprühen oder Bestreichen, also ein im Wesentlichen nur den Oberflächenbereich betreffendes Benetzen mit Zirbenöl, als auch ein Eintauchen oder Tränken zu verstehen, wobei das Zirbenöl in tiefere Schichten der Zirbenholzspäne eindringen kann. Die Zirbenholzspäne werden sozusagen als natürliches Speicherelement für das Zirbenöl eingesetzt, wobei die Späne die Duft- und Wirkstoffe gleichmäßig und lang andauernd abgeben.

Nach einem bevorzugten erfindungsgemäßen Verfahren zur Herstellung eines Zirbenholzpräparates werden vom Kernbereich eines Zirbenholzstückes dünne Schichten abgetragen, die zu den Spänen der gewünschten Größe zerkleinert werden. Die Schichtdicke beträgt vorzugsweise zwischen 1 mm und 0,01 mm, insbesondere ca. 0,1 mm. Die Schichtlänge kann bis zu einigen Metern betragen, welche dann zu kurzen Spänen mit Längen von 0,5 cm bis einigen Zentimetern zerkleinert werden. Durch Verarbeiten der Stämme zu Kantbrettern, Abtragen dünner Schichten durch Hobeln und Zerkleinern in feine Holzspäne durch Schneidmesser lassen sich feinstückige, voluminöse Holzmengen mit geringem Gewicht und großer Oberfläche herstellen, die besonders gut die Duft- und Wirkstoffe freisetzen können. Dabei wurde festgestellt, dass gerade im Kernholz in besonders hohem Maße die positiven Wirk- und Inhaltstoffe vorkommen. Das Kernholz umfasst das Stamminnere mit einem Durchmesser von 3 cm bis zu etwa 20 cm.

Ein besonders bevorzugtes erfindungsgemäßes Verfahren zum Herstellen eines Zirbenholzpräparates ist dadurch gekennzeichnet, dass das Zerkleinern der Späne mittels einer Vertikutiereinrichtung durchgeführt wird, durch welche ein Materialstrom mit dem Zirbelholzmaterial hindurchgeleitet wird. Vertikutierer, wie Häcksler, können Rotorblätter oder -flügel mit Schneidkanten aufweisen, durch welche Holz besonders schnell und fein zerkleinert werden kann. Durch ein schnell rotierendes Schneidmesser in Propellerform kann auch ein Luftstrom erzeugt werden, mit dem die abgehobelten dünnen Holzschichten angesaugt werden. Bei diesem Ansaugen kann auch ein Entfernen, insbesondere Abblasen oder Absaugen, von Holzstaub erfolgen.

Bei dem erfindungsgemäßen Verfahren zur Herstellung eines Zirbenholzpräparates ist es besonders vorteilhaft, dass in den Materialstrom Zirbenöl eingespritzt wird. Das Einsprühen von Zirbenöl geschieht am besten, wenn die Zirbenholzschichten aufgewirbelt werden. Hierdurch kann eine zuverlässige und gleichmäßige Benetzung erreicht werden.

Eine Variante des erfindungsgemäßen Verfahrens zur Herstellung eines Zirbenholzpräparates besteht darin, dass dem Materialstrom zusätzlich Wolle, insbesondere Schafwolle, zugeführt wird. Durch die Beigabe von Wolle bekommen Füllungen für z.B. Sitz- und Kopfkissen zusätzlich zur angenehmen Dufteigenschaft auch noch eine wärmende Komponente. Die Kombination von Spänen und Wolle wird als besonders behaglich empfunden, wobei etwa Lanolin in der Schafswolle sich auch auf den angenehmen Geruch positiv auswirkt.

Die Erfindung umfasst weiter ein Zirbenholzpräparat, welches Späne aus Zirbenholz aufweist, wobei die Späne mit Zirbenöl imprägniert sind. Das Zirbenholzpräparat kann insbesondere mit dem zuvor beschriebenen Verfahren hergestellt werden. Wie zuvor beschrieben, ergibt sich durch die Imprägnierung des dünn aufgeschnittenen Zirbenholzes mit Zirbenöl eine erhebliche Intensivierung der positiven Eigenschaften, insbesondere des Geruches hinsichtlich der Geruchsintensität und Geruchsdauer.

Weiter ist für das Zirbenholzpräparat erfindungsgemäß eine luftdurchlässige Aufnahme vorgesehen, in welcher die impränierten Späne aus Zirbenholz und gegebenenfalls die beigemischte Wolle angeordnet sind. Durch die luftdurchlässige Aufnahme für das Zirbenholzpräparat, insbesondere durch mit Zirbenholzpräparat gefüllte Stoffhüllen, kann sich der angenehme, wohltuende Duft ausbreiten. Als Aufnahme kann aber auch ein Netz oder ein starres Element, wie ein Gitterkasten vorgesehen sein, der einen guten Luftaustausch ermöglicht. Ein solcher Kasten kann etwa zur Anbringung an einem Heizkörper vorgesehen sein, wobei das Raumklima gut beeinflussbar ist.

Ferner ist gemäß der Erfindung die Aufnahme für das Zirbenholzpräparat als ein Säckchen, Kissen, Decke oder Matratze ausgebildet. In Versuchen hat sich gezeigt, dass Zirbenholz zur Erholung des Menschen beiträgt, insbesondere wenn in Schlafutensilien Zirbenholpräparat enthalten ist. Durch Zirbenholz kann bei Personen in körperlichen und mentalen Belastungssituationen eine niedrigere Herzfrequenzrate erreicht werden. Weiterhin kann der vegetative Erholungsprozess in einer Schlaf- oder Ruhephase beschleunigt und vierbessert werden.

Die Erfindung wird weiter anhand eines bevorzugten Ausführungsbeispieles erläutert, welches schematisch in der Zeichnung dargestellt ist. In der Zeichnung zeigen:

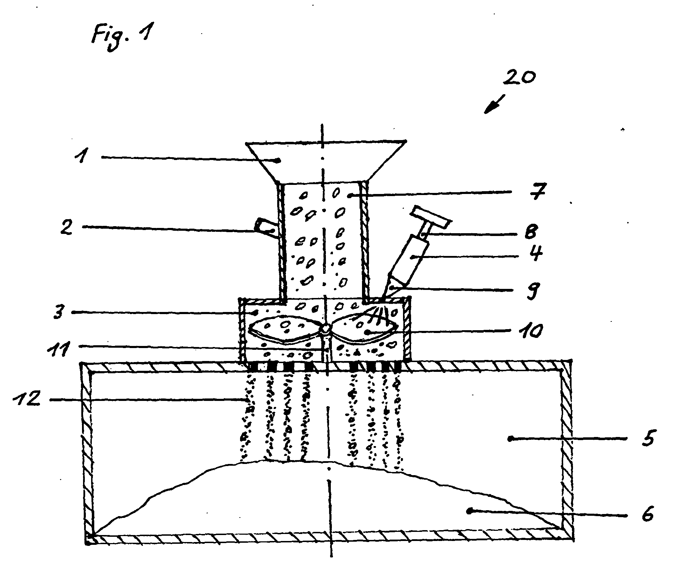

- Fig. 1: eine schematische, teilweise geschnittene Seitenansicht einer Vorrichtung für das erfindungsgemäße Verfahren zur Herstellung eines Zirbenholzpräparates.

Gemäß Fig. 1 umfasst eine Vorrichtung 20 für das erfindungsgemäße Verfahren zur Herstellung eines Zirbenholzpräparates einen Eingabetrichter 1, in welchen gehobelte Zirbenholzschichten und eventuell Schafwolle eingefüllt werden. Diese Materialien fallen als Materialstrom 12 durch ein Fallrohr 7 in die Vertikutiereinrichtung 3, wo sie durch mehrere an einem rotierenden Schaft 11 sitzende Flügelschneidblätter 10 zerkleinert werden.

Außerdem werden die halb zerkleinerten Materialien durch die rotierenden Flügelschneidblätter 10 aufgewirbelt. Diese Aufwirbelung ist einerseits erwünscht, um mittels einer Besprüheinrichtung 4 Zirbenöl zur gleichmäßigen Imprägnierung in den Materialstrom 12 aus Zirbenholzschichten und der Schafwolle einzuspritzen. Andererseits wird auch der beim Zerkleinerungsprozess anfallende feine Materialstaub durch die rotierenden Flügelschneidblätter 10 aufgewirbelt und kann über eine am Anschluss 2 angeschlossene, nicht dargestellte Absaugeinrichtung abgesaugt werden.

Die Besprüheinrichtung 4 kann dabei, wie in Fig. 1 dargestellt, einen gesteuert betätigten Kolben 8 oder eine Pumpe zum Einspritzen des Zirbenöles über eine Düse 9 aufweisen.

Durch Sieböffnungen zwischen Vertikutiereinrichtung 3 und einem unterhalb angeordneten Auffangbehälter 5 fällt der zerkleinerte, imprägnierte und mit Schafwolle vermischte Materialstrom 12 als Zirbenholzpräparat 6 in einen Auffangbehälter. Von dort kann es zur Auffüllung von luftdurchlässigen Aufnahmen, wie Säckchen, Kissen, Nackenrollen, Kniepolster, Sitzpolster, Decken, Duftkissen, Unterbetten oder Matratzen, entnommen werden kann. Das Zirbenholzpräparat kann auch zur Füllung von Baby- und Kinderkissen und -decken, welche kindgerecht geformt sein können, sowie von Plüschtieren und anderen Spielsachen eingesetzt werden. Auch am Körper zu tragende Stücke, wie gefüllte Gürtel für den Becken- und Nierenbereich oder Schals für den Hals- und Kopfbereich können mit dem erfindungsgemäßen Präparat hergestellt sein.

## Patentansprüche

1. Verfahren zur Herstellung eines Zirbenholzpräparates (6), wobei Holz der Zirbelkiefer (pinus cembra) zu Spänen zerkleinert wird, welche anschließend mit Zirbenöl imprägniert werden.

2. Verfahren nach Anspruch 1,
**dadurch gekennzeichnet,**
**dass** ein Kernbereich eines Zirbenholzstückes verwendet wird, von welchem dünne Schichten abgetragen werden, die zu den Spänen zerkleinert werden.

3. Verfahren nach Anspruch 1 oder 2,
**dadurch gekennzeichnet,**
**dass** das Zerkleinern zu Spänen mittels einer Vertikutiereinrichtung (3) durchgeführt wird, durch welche ein Materialstrom (12) mit dem Zirbenholzmaterial hindurchgeleitet wird.

4. Verfahren nach Anspruch 3,
**dadurch gekennzeichnet,**
**dass** in den Materialstrom (12) Zirbenöl eingespritzt wird.

5. Verfahren nach Anspruch 3 oder 4,
**dadurch gekennzeichnet,**
**dass** dem Materialstrom (12) Wolle, insbesondere Schafwolle, zugeführt wird.

6. Zirbenholzpräparat (6), welches Späne aus dem Holz der Zirbelkiefer (pinus cembra) aufweist, wobei die Späne mit Zirbenöl imprägniert sind.

7. Zirbenholzpräparat nach Anspruch 6,
**dadurch gekennzeichnet,**
**dass** den imprägnierten Spänen aus Zirbenholz Wolle, insbesondere Schafwolle, beigemischt ist.

8. Zirbenholzpräparat nach Anspruch 6 oder 7,
**dadurch gekennzeichnet,**
**dass** eine luftdurchlässige Aufnahme vorgesehen ist,
in welcher die imprägnierten Späne aus Zirbenholz und gegebenenfalls die beigemischte Wolle angeordnet sind.

9. Zirbenholzpräparat nach Anspruch 8,
**dadurch gekennzeichnet,**
**dass** die Aufnahme eine Textilie aufweist und insbesondere als ein Säckchen, Kissen, Nackenrolle, Kniepolster, Sitzpolster, Duftkissen, Decke, Unterbett, Matratze, Beckengürtel oder Schal ausgebildet ist.

## Claims

1. Method for producing a Swiss stone pine preparation (6), in which wood of Swiss stone pine (pinus cembra) is reduced to shavings which are subsequently impregnated with Swiss stone pine oil.

2. Method according to claim 1,
**characterized in that**
a core portion of a piece of Swiss stone pine is used, of which thin layers are taken off that are reduced to the shavings.

3. Method according to claim 1 or 2,
**characterized in that**
the reduction to shavings is carried out by means of a chopping device (3), through which a flow of material (12) containing the Swiss stone pine material is guided.

4. Method according to claim 3,
**characterized in that**
Swiss stone pine oil is injected into the flow of material (12) .

5. Method according to claim 3 or 4,
**characterized in that**
wool, in particular sheep's wool, is added to the flow of material (12).

6. Swiss stone pine preparation (6), which includes wood shavings of Swiss stone pine (pinus cembra), wherein the shavings are impregnated with Swiss stone pine oil.

7. Swiss stone pine preparation according to claim 6,
**characterized in that**
wool, in particular sheep's wool is mixed into the impregnated shavings of Swiss stone pine wood.

8. Swiss stone pine preparation according to claim 6 or 7,
**characterized in that**
an air-permeable receptacle is provided, in which the impregnated shavings of Swiss stone pine wood and, if applicable, the intermixed wool are arranged.

9. Swiss stone pine preparation according to claim 8,
**characterized in that**
the receptacle includes a textile and is designed in particular as a sachet, a cushion, a bolster, a knee pad, a seat pad, a scented cushion, a coverlet, a mattress cover, a mattress, a pelvic belt or a scarf.

## Revendications

1. Procédé de fabrication d'une préparation de pin cembro (6), du bois de pin cembro (ou pin arolle) *(pinus cembra)* étant fragmenté en copeaux qui sont ensuite imprégnés d'huile de pin cembro.

2. Procédé selon la revendication 1, ***caractérisé en ce qu*'**une zone de coeur d'un morceau de bois de pin cembro est utilisée, de laquelle on enlève des couches minces qui sont fragmentées en copeaux.

3. Procédé selon la revendication 1 ou 2, ***caractérisé en ce que*** la fragmentation en copeaux est réalisée au moyen d'un dispositif de verticutage (3) à travers lequel on fait passer un flux (12) de matériau contenant le matériau en bois de pin cembro.

4. Procédé selon la revendication 3, ***caractérisé en ce que*** de l'huile de pin cembro est injectée dans le flux (12) de matériau.

5. Procédé selon la revendication 3 ou 4, ***caractérisé en ce que*** de la laine, en particulier de la laine de mouton, est ajoutée au flux (12) de matériau.

6. Préparation (6) de bois de pin cembro qui comprend des copeaux de bois de pin cembro *(pinus cembra),* les copeaux étant imprégnés d'huile de pin cembro.

7. Préparation de pin cembro selon la revendication 6, ***caractérisée en ce que*** de la laine, en particulier de la laine de mouton, est mélangée aux copeaux de pin cembro imprégnés.

8. Préparation de pin cembro selon la revendication 6 ou 7, ***caractérisée en ce qu'***il est prévu un logement perméable à l'air, dans lequel sont placés les copeaux de pin cembro imprégnés et éventuellement la laine ajoutée.

9. Préparation de pin cembro selon la revendication 8, ***caractérisée en ce que*** le logement comporte un textile et est conformé en particulier en sachet, coussin, soutien de nuque, coussin de genou, coussin de siège, coussin odorant, couverture, sommier, matelas, ceinture de bassin ou châle.
